**Europäisches Patentamt**

(19) **European Patent Office**

**Office européen des brevets**

(11) Publication number: **0 147 975**

**A2**

(12) # EUROPEAN PATENT APPLICATION

(21) Application number: **84308612.5**

(22) Date of filing: **11.12.84**

(51) Int. Cl.⁴: **C 12 M 1/36**
**C 12 M 3/02**

(30) Priority: **28.12.83 JP 251788/83**

(43) Date of publication of application:
**10.07.85 Bulletin 85/28**

(84) Designated Contracting States:
**CH DE GB LI SE**

(71) Applicant: **AJINOMOTO CO., INC.**
**5-8, Kyobashi 1-chome, Chuo-ku**
**Tokyo 104(JP)**

(72) Inventor: **Shibai, Hiroshiro**
**No. 14-15, Wakamatsu-cho**
**Chigasaki-shi Kanagawa-ken(JP)**

(72) Inventor: **Nishimura, Naoki**
**No. 958, Kashimada, Saiwai-ku**
**Kawasaki-shi Kanagawa-ken(JP)**

(72) Inventor: **Kuratani, Takahiro**
**No. 1155-2, Nakamuruko Nakahara-ku**
**Kawasaki-shi Kanagawa-ken(JP)**

(72) Inventor: **Shimazaki, Koji**
**No. 958 Kashimada, Saiwai-ku**
**Kawasaki-shi Kanagawa-ken(JP)**

(74) Representative: **Bond, Bentley George et al,**
**Haseltine Lake & Co. 28 Southampton Buildings**
**Chancery Lane**
**London WC2A 1AT(GB)**

(54) **Method and apparatus for culture of animal cells.**

(57) There is described a method and apparatus for the culture of animal cells. The apparatus comprises a culture vessel (1) for culturing animal cells, gas supply means (21, 22, 23, 24) connected to the culture vessel (1) and provided with flow volume control means (17, 18, 19, 20) for controlling the flow volumes of at least nitrogen, oxygen, and carbon dioxide, flow volume measuring means (25, 26, 27, 28) for measuring the flow volumes of nitrogen, oxygen, and carbon dioxide, detection means (36, 37) for detecting the pH value and the dissolved oxygen concentration of the culture broth within the culture vessel (1), and a control circuit (50). The control circuit is adapted to receive signals from the measuring means (25, 26, 27, 28) and the detection means (36, 37), to increase or decrease the flow volume of carbon dioxide in response to the pH value signal thereby controlling the pH value to a prescribed level, to calculate the variations in the flow volumes of nitrogen and oxygen at the same time, necessary for compensating for the variation in the partial pressure of oxygen in the gaseous phase caused by the increase or decrease of the flow volume of carbon dioxide, to control the flow volume control means (17, 18, 19, 20) in accordance with the results of said calculation thereby to vary the flow volumes of nitrogen and oxygen, to calculate the flow volumes of nitrogen and oxygen necessary for controlling the dissolved oxygen concentration at a prescribed level in accordance with the dissolved oxygen concentration signal, and to control the flow volume control means (17, 18, 19, 20) in accordance with the results of said calculations thereby to vary the flow volume of carbon dioxide.

./...

# FIG.1

—|—

## METHOD AND APPARATUS FOR CULTURE OF ANIMAL CELLS

This invention relates to a method and apparatus for the culture of animal cells, and more particularly to the control of the dissolved oxygen concentration and the pH value of the culture broth in the culture of animal cells.

In the culture of animal cells, the control of the dissolved oxygen ($DO_2$) is generally carried out by varying the partial pressure of oxygen in the gaseous phase within the culture vessel. Specifically, when the $DO_2$ is higher than the set level, nitrogen ($N_2$) gas is fed to the gaseous phase to lower the partial pressure of oxygen in the gaseous phase and consequently lower the partial pressure of oxygen, or $DO_2$, in the culture broth. When the $DO_2$ is lower than the set level, oxygen ($O_2$) gas is fed to the gaseous phase to increase the partial pressure of oxygen in the gaseous phase and consequently increase the $DO_2$ in the culture broth. In accordance with this conventional method, however, the pH value of the culture broth is increased because the partial pressure of carbon

−2−

dioxide ($CO_2$) mixed in advance in the gaseous phase within the culture vessel for the control of pH value is lowered proportionately to the addition of nitrogen or oxygen.

The control of the pH value is generally carried out by increasing or decreasing the content of sodium hydrogencarbonate added in advance to the culture broth and the partial pressure of the $CO_2$ gas in the gaseous phase within the culture vessel. Specifically when the pH value is higher than the set level, $CO_2$ gas is fed to the gaseous phase to increase the partial pressure of $CO_2$ in the gaseous phase and consequently increase the partial pressure of $CO_2$ in the liquid phase proportionately and lower the pH value. When the pH value is higher than the set level, air, $O_2$ gas, $N_2$ gas, or a mixed gas thereof is fed to the gaseous phase to lower the partial pressure of $CO_2$ in the gaseous phase and increase the pH value. In accordance with this conventional method, however, the $DO_2$ level of the culture broth is inevitably affected because the variation in the partial pressure of $CO_2$ in the gaseous phase entails variation in the partial pressure of $O_2$.

In the conventional culture of animal cells, an attempt to control either the pH value or the $DO_2$ does not bring about any beneficial result because the two control systems interfere with each other as described above.

An object of the present invention is to provide a method and apparatus for culture which permit the control of the pH value and the control of the $DO_2$ value during the culture of animal cells to be carried out without entailing any mutual interference.

For the fulfilment of the object described above, this invention is constructed as follows.

Specifically, this invention concerns a method for the culture of animal cells by maintaining the pH value and the dissolved oxygen concentration in the culture broth within a culture vessel at respectively prescribed levels during the course of the culture of animal cells by continuing controlled supply of at least nitrogen, oxygen, and carbon dioxide into the gaseous phase within the culture vessel, which method is characterized by responding to any deviation of the pH value from the prescribed level by suitably increasing or decreasing the flow volume of carbon dioxide thereby correcting the deviation of the pH value and, at the same time, increasing or decreasing the flow volumes of nitrogen and oxygen thereby compensating for the variation of the partial pressure of oxygen in the gaseous phase caused by the increase or decrease of said flow volume of carbon dioxide and responding to any deviation of the dissolved oxygen concentration of the culture broth from the prescribed level by suitably increasing or decreasing the flow volumes of nitrogen and oxygen without varying the flow volume of carbon dioxide thereby correcting the deviation of the dissolved oxygen concentration of the culture broth, whereby the pH value and the dissolved oxygen concentration in the culture broth will be maintained at the prescribed levels.

This invention further concerns an apparatus for the culture of animal cells, comprising a culture vessel for culturing animal cells, gas supply means connected to the culture vessel and provided with flow volume control means for controlling flow volumes respectively of at least nitrogen, oxygen, and carbon dioxide, flow volume measuring means for measuring flow volumes of nitrogen, oxygen, and carbon dioxide, detection means for detecting the pH value and the dissolved oxygen concentration of the culture broth to be formed within the culture vessel, and control circuit means for receiving signals from the measuring means and the detection means, increasing or decreasing the flow volume of carbon dioxide in response to the pH value signal thereby controlling the pH value to a prescribed level and, at the same time, calculating the variations in the flow volumes of nitrogen and oxygen necessary for compesnating for any variation in the partial pressure of oxygen due to the increase or decrease of the flow volume of carbon dioxide, controlling the flow volume control means in accordance with the results of the calculation thereby varying the flow volumes of nitrogen and oxygen, calculating the flow volumes of nitrogen and oxygen necessary for controlling the dissolved oxygen concentration at a prescribed level in accordance with the dissolved oxygen concentration signal, and controlling the flow volume control means in accordance with the results of the calculation thereby varying the flow volume of carbon dioxide.

Here, the supply of oxygen to the culture vessel need not rely exclusively on oxygen gas but may rely on air in combination with oxygen gas or solely on air. For the sake of precautions, it is advantageous to effect the supply of oxygen normally depending preponderantly on air and, only when the maintenance of the dissolved oxygen concentration at the prescribed level soley by the air is difficult, add oxygen gas to the flow of air.

For a better understanding of the invention, reference will now be made, by way of example, to the drawings, in which:

Figure 1 is a schematic representation of one embodiment of an apparatus of the invention;

Figure 2 is a block diagram illustrating the operation of the control circuit of the apparatus of Figure 1;

Figures 3a and 3b together show a typical program flow chart applicable to the apparatus shown in Figures 1 and 2;

Figures 4 and 5 are graphs which are referred to in Experiments 1 and 2 below.

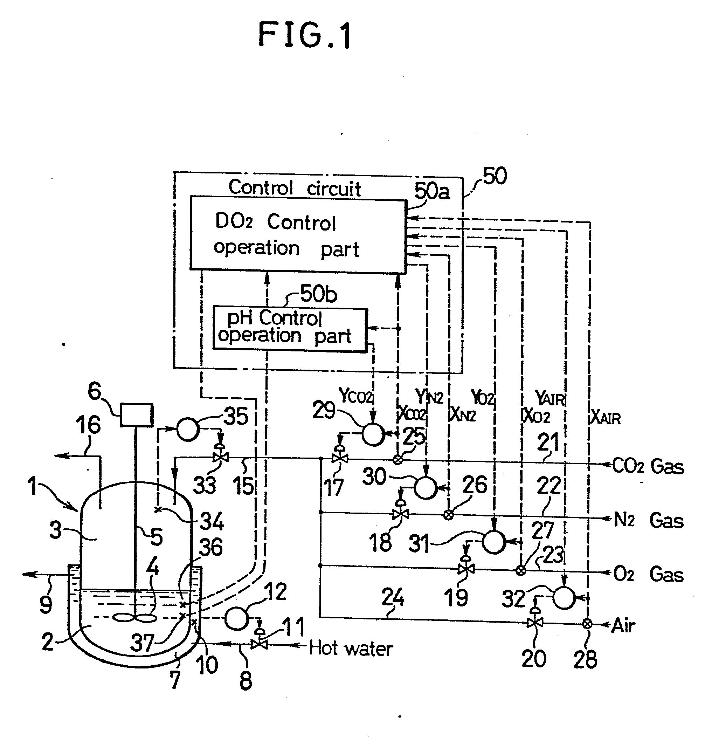

Fig. 1 is a schematic diagram of the control system in an apparatus for the culture of animal cells as one embodiment of this invention. With reference to Fig. 1, a culture vessel 1 contains culture broth 2. Above the culture broth 2, a gaseous phase 3 formed of a mixed gas of carbon dioxide, oxygen, nitrogen, and air is formed inside the culture vessel 1. This vessel 1 is provided with agitation blades 4 disposed so as to occupy a position within the culture broth 2. These agitation blades are slowly driven to agitate the culture broth by a motor 6 to which the agitation blades 4 are connected through the medium of a drive shaft 5 extended upwardly thence. This agitation accelerates the passage of the gas from the gaseous phase 3 into the culture broth. The pH value and the dissolved oxygen concentration of the culture broth are affected by the partial pressure of carbon dioxide and that of oxygen in the gaseous phase 3. A hot water jacket 7 is formed on the lower half periphery of the vessel 1. This hot water jacket 7 is provided with a hot water feed pipe 8 and a water discharge pipe 9 to permit circulation of hot water through the jacket 7. A temperature sensor 10 is disposed inside the jacket 7 and a flow volume control valve 11 is disposed inside the hot water feed pipe 8. The signal from the temperature sensor 10 is forwarded to a flow volume control circuit 12 and the output from this control circuit 12 effects proportional control of the flow volume control valve 11 so as to maintain the temperature of the hot water within the jacket 7 in a required range.

A mixed gas feed pipe 15 and a gas discharge pipe 16 open into the gaseous pahse 3 inside the vessel 1. The mixed gas feed pipe 15 is connected to a $CO_2$ feed pipe 21, a $N_2$ feed pipe

22, an $O_2$ feed pipe 23, and an air feed pipe 24 provided respectively with flow volume control valves 17, 18, 19, and 20.

The feed pipes 21, 22, 23, and 24 are provided respectively with flow meters 25, 26, 27, and 28 and adapted to issue flow volume signals $X_{CO2}$, $X_{N2}$, $X_{O2}$, and $X_{AIR}$. The flow volume control valves 17, 18, 19, and 20 are provided with valve drive circuits 29, 30, 31, and 32 respectively. By the outputs from these circuits, the respective valves are driven in an opening direction or closing direction. A flow volume control valve 33 is disposed in the mixed gas feed pipe 15 and a pressure gauge 34 is disposed in the gaseous phase of the vessel 1. The pressure signal issuing from the pressure gauge 34 is fed in a flow volume control circuit 35. This circuit 35, by virtue of its output, controls the flow volume control valve 33 to maintain the pressure of the gaseous phase 3 substantially at a fixed level. Inside the culture broth 2, a dissolved oxygen concentration detector 36 and a pH detector 37 are disposed.

A control circuit 50 is installed for the purpose of controlling the opening degrees of the flow volume control valves 17, 18, 19, and 20. The control circuit 50 maybe formed of a micro-computer. In terms of function, it may well be regarded as comprising a $DO_2$ control operation part 50a for effecting the control of the dissolved oxygen concentration and a pH control operation part 50b for effecting the control of the pH value. The $DO_2$ control operation part 50a admits the $DO_2$ signal from the dissolved oxygen concentration detector 36 and also the flow volume signals $X_{CO2}$, $X_{N2}$, $X_{O2}$, and $X_{AIR}$ from the flow volume meters 25, 26, 27, and 28. The pH control operation part 50b admits the flow volume signal $X_{CO2}$ from the flow volume meter 25 and the pH signal from the pH detector 37. The output of the pH control operation part 50b is given to the valve drive circuit 29 on one hand and fed in the $DO_2$ control operation part 50a on the other hand. The output of the $DO_2$ control operation part 50a is given to the valve drive circuits 30, 31, and 32.

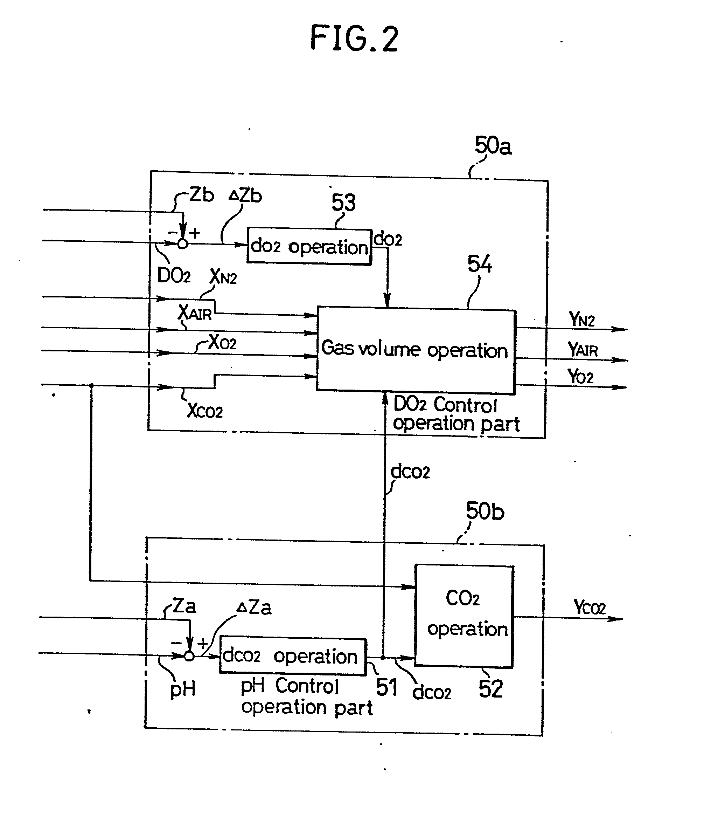

Fig. 2 is a functional block diagram for illustrating by model the operation of the control circuit 50. The pH control operation part 50b compares the detected pH value brought in from the pH detector 37 with the set pH value and, based on the difference, gives rise to a $CO_2$ flow volume correction signal $d_{CO2}$. In the present embodiment, the $CO_2$ flow volume correction signal $d_{CO2}$ is formed as follows. Let the formula:

$$F = X_{CO2} + X_{N2} + X_{O2} + X_{AIR} \qquad (1)$$

define the total amount, F, of the flow volumes, $X_{CO2}$, $X_{N2}$, $X_{O2}$, and $X_{AIR}$ of carbon dioxide, nitrogen, oxygen, and air to be fed to the gaseous phase 3 of the culture vessel 1, Za stand for the prescribed pH value, and $\Delta Za$, the difference between the detected pH value and the prescribed pH value, to take a proper numerical value in the range of 0.05 to 0.1 such as, for example:

$|\Delta Za| > 0.05 \sim 0.1$, and then the following relation will be satisfied.

$$d_{CO2} = -\alpha F \quad \text{(Prescribed pH value > detected pH value)} \quad (2)$$

$$d_{CO2} = \alpha F \quad \text{(Prescribed pH value < detected pH value)} \quad (3)$$

(wherein $\alpha$ stands for a certain numerical value in the range of 0.01 to 0.05).

When the difference, $\Delta Za$, falls in the range of $|\Delta Za| \leq$ 0.05 to 0.1, it is presumed that the following relation is satisfied.

$$d_{CO2} = 0 \qquad\qquad (4)$$

This arithmetic operation is carried out in the $d_{CO2}$ operation part 51 of the pH control operation part 50b.

The $CO_2$ flow volume correction signal $d_{CO2}$ thus formed is used in the arithmetic operation for the calculation of required carbon dioxide flow volume $Y_{CO2}$. This arithmetic operation is carried out in accordance with the following formula in the $Y_{CO2}$ operation part 52.

$$Y_{CO2} = X_{CO2} + d_{CO2} \qquad\qquad (5)$$

The signal of the required carbon dioxide flow volume $Y_{CO2}$ thus obtained is given as the output from the pH control operation part 50b to the valve drive circuit 29.

Further, the $CO_2$ flow volume correction signal $d_{CO2}$ is forwarded from the pH control operation part 50b to the $DO_2$ control operation part 50a. The $DO_2$ control operation part 50a admits the $DO_2$ signal from the $DO_2$ detector 36 and compares

it with the $DO_2$ set value Zb and calculates the value of $d_{02}$ in accordance with the formula:

$$d_{02} = \beta F \quad (\text{Required } DO_2 \text{ value} > \text{Detected } DO_2 \text{ value}) \quad (6)$$

$$d_{02} = -\beta F \quad (\text{Required } DO_2 \text{ value} < \text{Detected } DO_2 \text{ value}) \quad (7)$$

(wherein $\beta$ stands for a certain value in the range of 0.01 to 0.05), where the value of the difference $\Delta Zb$ is a certain value in the range of 0.005 to 0.02 atm, i.e. $|\Delta Zb| \geq 0.005 \sim 0.02$, or in accordance with the formula:

$$d_{02} = 0 \qquad\qquad\qquad\qquad (8)$$

where the value of the difference $\Delta Zb$ is a certain value in the range of $|\Delta Zb| \leq 0.005 \sim 0.02$. This arithmetic operation is carried out in the $d_{02}$ operation part 53. The $d_{02}$ and $d_{CO2}$ signals are both fed in the flow volume operation part 54. The operation part 54 admits the flow volume signals $X_{CO2}$, $X_{N2}$, $X_{02}$, and $X_{AIR}$ from the flow volume meters 25, 26, 27, and 28 and performs the following arithmetic operations.

First on the assumption that air is preferentially used over oxygen, the operation is examined to determine whether or not air alone is sufficient for the supply of oxygen required for the culture. Specifically, the amount of oxygen required is the sum of the current flow volume $X_{02}$ of oxygen, the partial pressure of oxygen or $(1/5)X_{AIR}$ contained in the flow volume $X_{AIR}$ of air, and the corrected $O_2$ flow volume $d_{02}$. If this sum is equal to or smaller than 1/5 of the total volume F of all the gases minus the carbon dioxide portion, the flow volume of air being supplied may be increased or decreased suitably to ensure required supply of oxygen. The condition is expressed by the following formula.

$$(1/5) \{F - (X_{CO2} + d_{CO2})\} \geqq X_{02} + (1/5)X_{AIR} + d_{02} \qquad (9)$$

From the formula (9), the following formula can be derived.

$$5 d_{02} + d_{CO2} \leqq F - (X_{CO2} + 5 X_{02} + X_{AIR}) \qquad (10)$$

Since the required supply of oxygen is wholly obtained from air alone here, the target oxygen flow volume $Y_{02}$ can be set at 0. Further from the balance of the amount of oxygen, the following formula is obtained.

$$Y_{AIR} = 5 (X_{02} + d_{02}) + X_{AIR} \qquad (11)$$

Then, from the balance of the total amount of gases, the following formula is derived.

$$Y_{N2} = F \{5 (X_{02} + d_{02}) + X_{AIR} + X_{CO2} + d_{CO2}\} \qquad (12)$$

The $DO_2$ control operation part 50a carries out the determination by the formula (9) in the operation part 54. When the formula (9) is satisfied, it carries out the arithmetic operation in accordance with the formula (11) and the formula (12) to form the target air flow volume signal $Y_{AIR}$ and the target nitrogen flow volume signal $Y_{N2}$ and issues these signals respectively to the valve drive circuits 32, 30. As the result, the flow volume control valves 18, 20 are adjusted to respectively required opening degrees. The flow volume control valve 19 is closed by the signal from the drive circuit 31. The formula (12) represents the total amount of gases required for maintaining the pressure within the culture vessel 1 at a fixed level minus the flow volume of carbon dioxide found by the formula (5) and the flow volume of air found by the formula (11). In other words, the aforementioned control consists in first determining the flow volume of carbon dioxide required on the

—12—

basis of the value of correction in the $CO_2$ flow volume, then determining the flow volume of air so as to maintain the partial pressure of oxygen in the gaseous phase 3 at a prescribed level, and correcting the flow volume of nitrogen accordingly. When the value of correction in the $CO_2$ flow volume is 0, namely when the pH value remains within the prescribed range, the air flow volume $Y_{O2}$ and the nitrogen flow volume $Y_{N2}$ required are determined in accordance with the $DO_2$ signal without causing any variation in the flow volume of carbon dioxide.

If the condition defined by the formula (9) is not satisfied, namely when the required supply of oxygen is not obtained only by air, the formula of condition corresponding to the formula (9) will be as follows.

$$(1/5) \left\{ F - (X_{CO2} + d_{CO2}) \right\} < X_{O2} + (1/5)X_{AIR} + d_{O2} \qquad (13)$$

This formula of condition can be rearranged as follows.

$$5 \, d_{O2} + d_{CO2} > F - (X_{CO2} + 5 \, X_{O2} + X_{AIR}) \qquad (14)$$

Since the inner pressure can be controlled by adjusting the feed ratio of air and oxygen, the supply of nitrogen may well be regarded as unnecessary. Thus, the relation $Y_{N2} = 0$ is presumed. Under this particular condition, the following relation is satisfied for the maintenance of the pressure at a fixed level.

$$Y_{O2} + Y_{AIR} = F - (X_{CO2} + d_{CO2}) \qquad (15)$$

From the balance of the amount of oxygen supplied, the following formulas are derived.

$$Y_{O2} = 1/4 \left\{ 5 \, (X_{O2} + d_{O2}) + X_{AIR} + X_{CO2} + d_{CO2} - F \right\} \qquad (16)$$

$$Y_{AIR} = 1/4 \left\{ 5 \, (F - X_{O2} - X_{CO2} - d_{O2} - d_{CO2}) - X_{AIR} \right\} \qquad (17)$$

The $D_{O2}$ control operation part 50a carries out the determination by the formula (13), presumes $Y_{N2} = 0$ when the formula (13) is satisfied, then performs the operations in accordance with the formulas (16) and (17) to determine the required oxygen flow volume $Y_{O2}$ and the required air flow volume $Y_{AIR}$, gives corresponding signals to the valve drive circuits 31, 32, and controls the valves 19, 20. Of course, the valve 18 is closed at this time. It is, of course, possible to obtain required supply of oxygen by varying the supply ratio of oxygen and nitorgen without effecting supply of air. The formula for the determination of this supply ratio can be easily derived from the balance of the feed amount of oxygen on the assumption that the inner pressure is fixed.

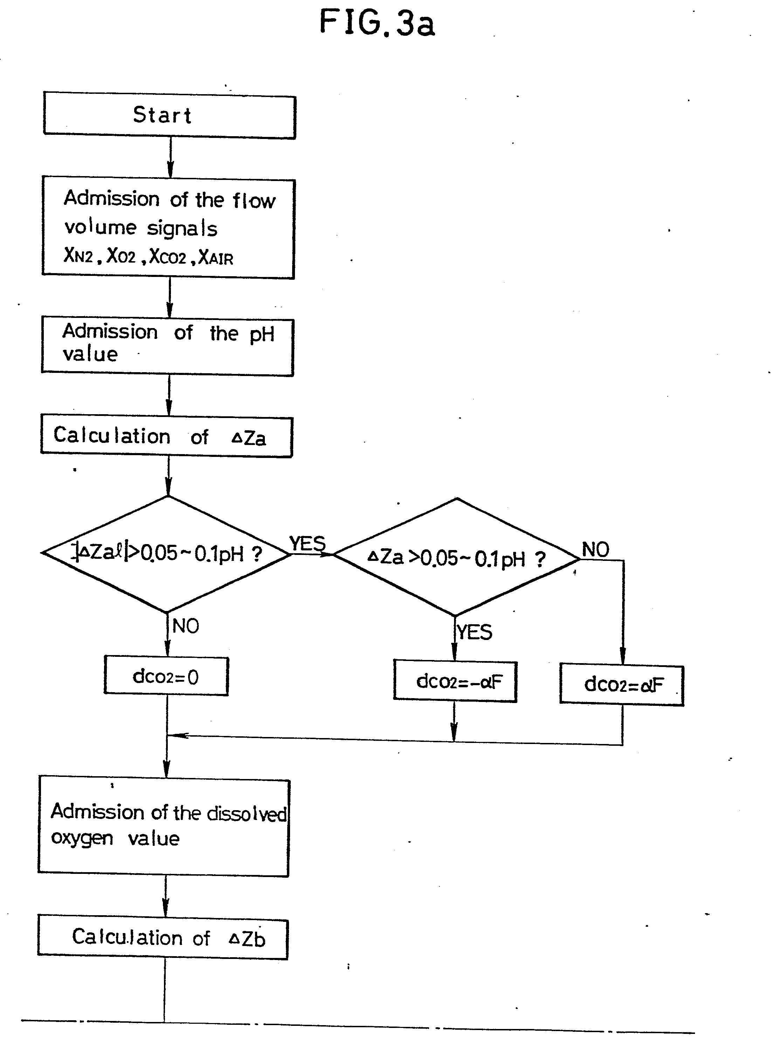

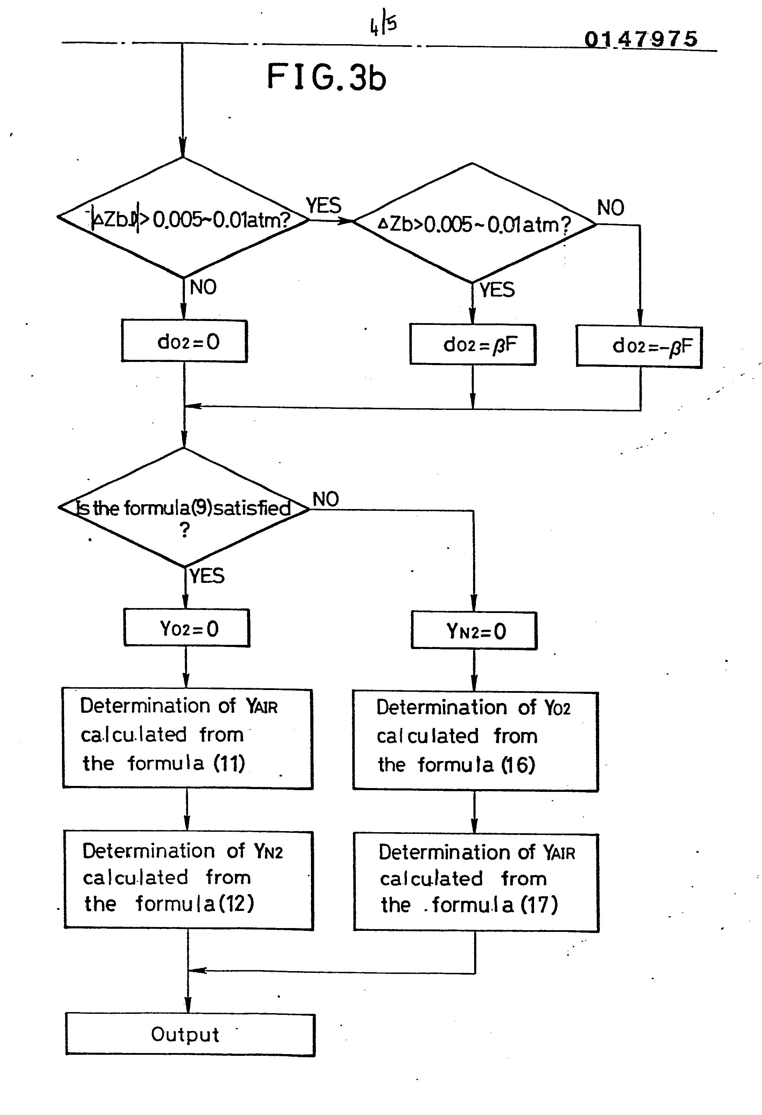

Fig. 3 represents a typical program flow chart of the embodiment described above with reference to Fig. 1 and Fig. 2. The conditions of the culture broth 2 and the gaseous phase 3 within the culture vessel 1 can be properly controlled by incorporating into the microprocessor forming the control device 50 a program required for materializing this flow and executing this program at suitable intervals such as, for example, intervals of 60 seconds.

The diagram depicts a method for the feed of gas to the gaseous phase. In the high-density culture (with the number of cells exceeding $1 \times 10^6$/ml, generally falling in the range of $1 \times 10^7$/ml to $1 \times 10^8$/ml), desired uniform control of the pH value and the $DO_2$ in the culture broth can be obtained by using a gas-pervious membrane such as silicone tube.

This gas-pervious membrane can be used in accordance with the method disclosed in Japanese Patent Application Laid-open SHO 58(1983)-116678, with necessary modifications.

The present invention can be used on all the animal cells which are grown by the single-layer culture method and the suspension culture method.  For example, it suits culture of:

a)  fibroblasts such as Balb 3T3 Clone A31 (Aaronson, S.A. & Todaro, G.T., J. Cell Physoil., 72, 141 (1968)), CCRF-S-180II (Faley, G., et al., Cancer Res., 20, 930 (1960)), WI-38 (Hayflick, L., Exp, Cell Res., 37, 614 (1965)), L929 (Stanford, K.K., et al., F. Nat. Cancer Inst., 9, 229 (1948)), CV-1 (Jensen, F.C., et al., Pro. N.a.s., 52, 53 (1964)), and BHK-21 Clone 13 (Macpharson, I.A. & Stoclcer, M.G.P., J. Nat. Cancer Inst., 30, 795 (1963)).

b)  epithelial cells such as Chang Liver (Change, R.S. PSEBM, 87, 40 (1954)), FL. (Fogh, J. & Lund, R., PSEBM, 94, 532 (1957)), Hela (Gey, G.O., et al., J. Exp. Med., 97, 695 (1953)), Y-1 (Yasumura, Y. et al., Cancer Res., 26, 529 (1966)) and BS-C-1 (Hopps, H.E., et al., J. Immunol., 91, 416 (1963)).

c)  limphoblasts such as RPMI 1788 (Moore, G.E., et al., J. Nat. Cancer Inst., 43, 1119 (1969)), CCRF-CEM (Foley, G.E., et al., Cancer.,18,552(1965)), EB-3 (Epstein, M.A. & Earr, Y. M., Lancet. i, 252 (1964), and MPC-11 (Laslcov, R & Scharff, M.D., J. Exp. Med., 131, 515 (197))

-15-

Examples of the culture medium advantageously usable for the present invention are E-MEM (Eagle, H. Science, <u>130</u>, 432 (1959)), D-MEM (Dulbecco, R. and Freeman, G., Virology, <u>8</u>, 396 (1959)), RPMI-1640 (Moore, G.E., et al., J.A.M.A., <u>199</u>, 519 (1967)), and RITC55-9 (Yamane, I. et al., Exp. Cell Res., <u>134</u>, 470 (1981)).

(Effect of the Invention)

In accordance with this invention, the pH value and the dissolved oxygen concentration of the culture system are kept under constant measurement and, upon detection of a deviation of the pH value from the prescribed level, the correction of this deviation of the pH value is effected by suitably increasing or decreasing the flow volume of carbon dioxide and, in anticipation of the variation in the partial pressure of oxygen in the gaseous phase due to the increase or decrease of the flow volume of carbon dioxide, the compensation for this variation is effected by suitably increasing or decreasing the flow volume of nitrogen and oxygen. Then, on detection of a deviation of the dissolved oxygen concentration from the prescribed level, the correction of this deviation of the dissolved oxygen concentration is effected by suitably increasing or decreasing the flow volumes of nitrogen and oxygen and, in anticipation of the variation caused in the partial pressure of the carbon dioxide in the gaseous phase by the increase or decrease of the flow volumes of nitrogen and oxygen, the compensation for the variation is effected by suitably increasing or decreasing the flow volume of carbon dioxide. As the result, the disadvantage of the conventional method and apparatus that when either of the pH value and the dissolved oxygen concentration is controlled, the control itself is affected by the other factor is eliminated.

Experiment 1:

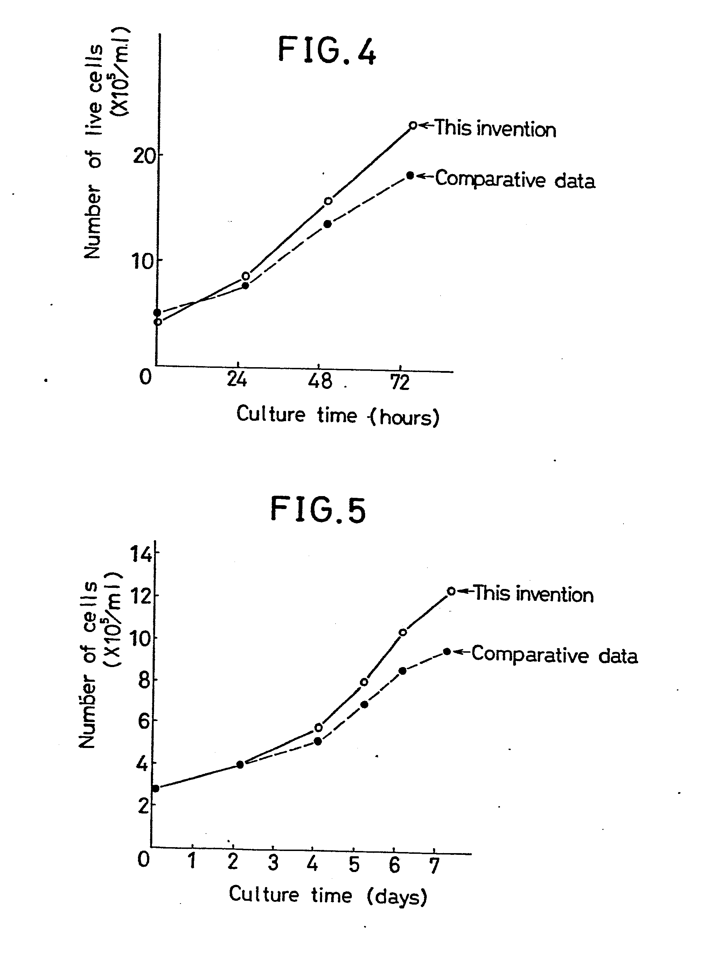

In 1,000 ml of RITC 55-9 medium, limphoblasts originating in human umbilican hemolymph cells (Sato, T. et al, Exp. Cell Res., 138, 127-134 (1982)) were suspended in a concentration of $4 \sim 5 \times 10^5/ml$. In a spinner flask of glass (made by Bellco Inc.) having an inner volume of 1,000 ml, the suspension was cultured as agitated at a rate of 150 rpm. At this time, the pH value of the suspension was controlled at $7.2 \pm 0.05$ and the dissolved oxygen concentration ($DO_2$) at $0.05 \pm 0.02$ atm by the use of the apparatus of this inveniton. For comparison, the culture was carried out by the method of feeding the gaseous phase of the spinner flask with a mixed gas of air containing 5% carbon dioxide gas without the use of the apparatus of this invention. In both the experiments, the gas flow volume fixed at 200 ml/min at the exhaust gas outlet of the culture vessel. During the culture, the culture broth was sampled at intervals in a volume of 1 to 2 ml and tested for number of live cells. The results are shown in Fig. 4.

Experiment 2:

In 100 ml of D-MEM medium containing 10% FBS' (blood serum of bovine infant), cells, Balb/3T3 $SV_{40}$ transformed (Todaro,

G. T., Science, 162, 1024 (1968)), obtained by transforming cells Balb 3T3 originating in a murine infant with virus $SV_{40}$

were suspended in a concentration of 4 ~ 5 x $10^5$/ml. This

suspension was added to 900 ml of 10% FBS' D-MEM medium containing 4 g of Cytodex 1 (made by Farmacia Inc.) per liter.

In a micro-carrier flask of glass having an inner volume of 100 ml (made by Bellco Inc.), the resultant mixture was cultured as agitated at a rate of 50 rpm.

At this time, the apparatus of this invention was used to control the pH value at 7.0 ± 0.05 and the $DO_2$ at 0.10 ± 0.02 atm. For comparison, the culture was carried out by the method of feeding   the gaseous phase of the micro-carrier flask with a mixed gas of air containing 5% carbon dioxide gas without the use of the apparatus of this inveniton. In both the experiments, the gas flow volume was fixed at 100 ml/min at the  exhaust gas  outlet of the culture vessel. During the culture, the culture broth was sampled at intervals in a volume of 1 to 2 ml and tested for number of cells by the nuclear dying method. The results are shown in Fig. 5.

Claims

1.  A method for the culture of animal cells by maintaining the pH value and the dissolved oxygen concentration in the culture broth within a culture vessel at respectively prescribed levels during the course of said culture of animal cells by continuing controlled supply of at least nitrogen, oxygen, and carbon dioxide into the gaseous phase within said culture vessel, which method is characterized by

(a) responding to any deviation of the pH value from said prescribed level by suitably increasing or decreasing the flow volume of carbon dioxide thereby correcting said deviation of the pH value,

(b) increasing or decreasing the flow volumes of nitrogen and oxygen at the same time, thereby compensating for the variation in the partial pressure of oxygen in the gaseous phase caused by said increase or decrease of the flow volume of carbon dioxide,

(c) and responding to any deviation of the dissolved oxygen concentration of the culture broth from said prescribed level by suitably increasing or decreasing the flow volumes of nitrogen and oxygen without varying the flow volume of carbo dioxide thereby correcting said deviation of the dissolved oxygen concentration of the culture broth,

(d) whereby the pH value and the dissolved oxygen concentration in the culture broth will be maintained at said prescribed levels.

2. A method according to Claim 1, wherein said oxygen to be supplied is in the form of air or air in combination with oxygen gas.

3. A method according to Claim 1 or Claim 2, which further comprises controlling the inner pressure of said culture vessel to a prescribed level.

4. An apparatus for the culture of animal cells, comprising

(A) a culture vessel for culturing animal cells,

(B) gas supply means connected to said culture vessel and provided with flow volume control means for controlling flow volume respectively of at least nitrogen, oxygen, and carbon dioxide,

(C) flow volume measuring means for measuring flow volumes of nitrogen, oxygen, and carbon dioxide,

(D) detection means for detecting the pH value and the dissolved oxygen concentration of the culture broth to be formed within said culture vessel,

(E) and control circuit means for

(a) receiving signals from said measuring means and said detection means,

(b) increasing or decreasing the flow volume of carbon dioxide in response to the pH value signal thereby controlling the pH value to a prescribed level,

(c) calculating the variations in the flow volumes of nitrogen and oxygen at the same time, necessary for compensating for the variation in the partial pressure of oxygen in the gaseous phase caused by said increase or decrease of the flow volume of carbon dioxide,

(d) controlling said flow volume control means in accordance with the results of said calculation thereby varying the flow volumes of nitrogen and oxygen,

(e) calculating the flow volumes of nitrogen and oxygen necessary for controlling said dissolved oxygen concentration at a prescribed level in accordance with the dissolved oxygen concentration signal,

(f) and controlling said flow volume control means in accordance with the results of said calculation thereby varying the flow volume of carbon dioxide.

5. An apparatus according to Claim 4, wherein said gas supply means is provided with oxygen and air supply means and the oxygen content in the gaseous phase is maintained by supply of air or air in combination with oxygen gas.

6. An apparatus according to Claim 4 or Claim 5, which further comprises an inner pressure control device for maintaining the inner pressure of said culture vessel at a prescribed level.

# FIG.1

# FIG.2

## FIG.3a

```
┌─────────────────────────┐
│          Start          │
└─────────────────────────┘
             │
             ▼
┌─────────────────────────┐
│  Admission of the flow  │
│  volume signals         │
│  X_N2, X_O2, X_CO2, X_AIR│
└─────────────────────────┘
             │
             ▼
┌─────────────────────────┐
│  Admission of the pH    │
│  value                  │
└─────────────────────────┘
             │
             ▼
┌─────────────────────────┐
│  Calculation of  ΔZa    │
└─────────────────────────┘
             │
             ▼
```

$|\Delta Za| > 0.05 \sim 0.1\,pH\ ?$ —— YES —— $\Delta Za > 0.05 \sim 0.1\,pH\ ?$ —— NO

NO         YES

$d_{CO_2} = 0$      $d_{CO_2} = -\alpha F$      $d_{CO_2} = \alpha F$

```
┌─────────────────────────┐
│ Admission of the dissolved│
│ oxygen  value           │
└─────────────────────────┘
             │
             ▼
┌─────────────────────────┐
│  Calculation of  ΔZb    │
└─────────────────────────┘
```

0147975

# FIG.3b

$|\Delta Zb| > 0.005 \sim 0.01 \text{atm}?$ — YES → $\Delta Zb > 0.005 \sim 0.01 \text{atm}?$ — NO

NO ↓

$d_{O2} = 0$

YES ↓

$d_{O2} = \beta F$

$d_{O2} = -\beta F$

Is the formula(9)satisfied? — NO

YES ↓

$Y_{O2} = 0$

$Y_{N2} = 0$

Determination of $Y_{AIR}$ calculated from the formula (11)

Determination of $Y_{O2}$ calculated from the formula (16)

Determination of $Y_{N2}$ calculated from the formula(12)

Determination of $Y_{AIR}$ calculated from the formula (17)

Output

0147975

5/5

# FIG. 4

Number of live cells ($\times 10^5$/ml)

o← This invention

●← Comparative data

20

10

0    24    48    72

Culture time (hours)

# FIG. 5

Number of cells ($\times 10^5$/ml)

14

12    o←This invention

10

8    ●←Comparative data

6

4

2

0    1    2    3    4    5    6    7

Culture time (days)